# EUROPÄISCHE PATENTSCHRIFT

(11) **EP 1 394 149 B1**
(45) Veröffentlichungstag und Bekanntmachung des Hinweises auf die Patenterteilung: **19.01.2005**
(21) Anmeldenummer: 03024787.8
(22) Anmeldetag: 13.01.2000
(51) Int. Cl.: C07D 213/50, C07D 213/57, C07D 213/52

(54) **Verfahren zur Herstellung von 1-(6-Methylpyridin-3-yl)-2-[4-(methylsulfonyl)-phenyl]ethanon**
Process for the preparation of 1-(6-Methylpyridin-3-yl)-2-[4-(methylsulfonyl)-phenyl]ethanon
Procédé de préparation de 1-(6-Methylpyridin-3-yl)-2-[4-(methylsulfonyl)-phenyl]ethanon

(30) Priorität: 14.01.1999 EP 99100590
(43) Veröffentlichungstag der Anmeldung: 03.03.2004
(62) Teilanmeldung aus: 00901555.3
(73) Patentinhaber: Lonza AG, 4002 Basel (CH)
(72) Erfinder: Armbruster, Erich, 3904 Naters (CH); Bessard, Yves, 3960 Sierre (CH); Kuo, David, New Jersey 07078 (US); Leresche, James, 3930 Visp (CH); Proplesch, Ralf, 5063 Wölflinswil (CH); Roduit, Jean-Paul, 1950 Sion (CH)

(56) Entgegenhaltungen:
- WO-A-98/47871
- WO-A-99/15503
- WO-A-99/55830
- BADIA D ET AL: "UNSYMMETRICALLY SUBSTITUTED DEOXYBENZOINS; AN IMPROVED PREPARATIVE ROUTE" BULLETIN DES SOCIETES CHIMIQUES BELGES,GB,PERGAMON PRESS, Bd. 98, Nr. 1, 1989, Seiten 77-81, XP000886342
- BRAY B L ET AL: "SYNTHESIS OF ACYLPYRROLES VIA ALPHA-(DIMETHYLAMINO)-ALPHA-PYRROLYLACETON ITRILES" JOURNAL OF ORGANIC CHEMISTRY,US,AMERICAN CHEMICAL SOCIETY, Bd. 53, Nr. 26, 1988, Seiten 6115-6118, XP000886363

## Beschreibung

Die Erfindung betrifft die Verbindung 1-(6-Methylpyridin-3-yl)-2-[(4-(methylsulfonyl)-phenyl]ethanon der Formel 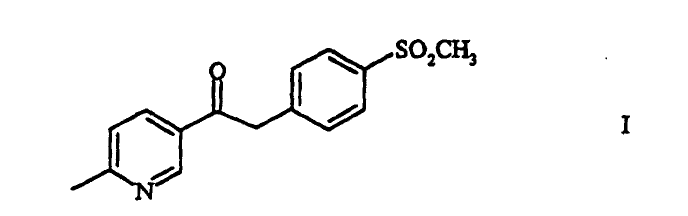 sowie ein Verfahren zu ihrer Herstellung.

1-(6-Methylpyridin-3-yl)-2-[(4-(methylsulfonyl)phenyl]ethanon ist ein neues Ausgangsprodukt zur Herstellung von sogenannten COX-2-Inhibitoren, pharmazeutischen Wirkstoffe mit schmerz- und entzündungshemmender Wirkung (R.S. Friesen et al., Bioorganic & Medicinal Chemistry Letters 8 (1998) 2777 - 2782; WO 98/03484).

Die Aufgabe der vorliegenden Erfindung bestand darin, ein neues Ausgangsprodukt zur Herstellung pharmazeutisch wirksamer Verbindungen zur Verfügung zustellen. Eine weitere Aufgabe bestand darin, ein technisch gangbares Verfahren zur Herstellung dieses Ausgangsprodukts bereitzustellen.

Diese Aufgabe wurde mit der Verbindung nach Anspruch 1 und dem erfindungsgemässen Verfahren nach Anspruch 2 gelöst.

Das erfindungsgemässe Verfahren stellt in einer besonders vorteilhaften Ausführungsform die letzte Stufe eines vierstufigen Verfahrens dar, wobei
in einer ersten Stufe a) 2-Methyl-5-ethylpyridin bei 500°C bis 700°C in Gegenwart eines Katalysators in das 2-Methyl-5-vinylpyridin überführt wird,
in einer zweiten Stufe b) das 2-Methyl-5-vinylpyridin mit Ozon und anschließend durch Reduktion in den 2-Methylpyridin-5-carbaldehyd überführt wird,
in einer dritten Stufe c) der 2-Methylpyridin-5-carbaldehyd mit einem Dialkylamin und einer Cyanverbindung in das entsprechende N,N-Dialkylamino-(6-methyl-3-pyridyl)acetonitril überführt wird, und schliesslich
in einer letzten Stufe d) das N,N-Dialkylamino-(6-methyl-3-pyridyl)acetonitril in Gegenwart einer Base mit einem 4-(Methylsulfonyl)benzylhalogenid zum 1-(6-Methylpyridin-3-yl)-2-[(4-(methylsulfonyl)phenyl]ethanon als Endprodukt umgesetzt wird.

Ein beträchtlicher Vorteil dieser Ausführungsform des erfindungsgemässen Verfahrens beruht auf der Tatsache, dass vom grosstechnisch verfügbaren 2-Methyl-5-ethylpyridin ausgegangen werden kann.

### Stufe a):

Die Dehydrierung von 2-Methyl-5-ethylpyridin zum 2-Methyl-5-vinylpyridin ist literaturbekannt (z.B. A. Nenz et al., Hydrocarbon Processing, 47(11), 1968, 139-144; US-A-2,769,773).

Die Umsetzung verläuft bei 500°C bis 700°C, vorzugsweise bei 600°C bis 700°C, in Gegenwart einer Vielzahl von verschiedenen Katalysatoren. In der Regel gelangen Katalysatoren auf Basis von Siliciumdioxid, Silicagel, Eisenoxid, Zinkoxid, Chromoxid, Kupferchromit, Magnesiumoxid, Kaliumoxid, Aluminiumoxid oder Borphosphat, einzeln oder als Mischung, gegebenenfalls aufgebracht auf einen Träger zum Einsatz. Gute Resultate lassen sich u.a mit einem Zinkoxidkatalysator aufgebracht auf Bimsstein als Träger erzielen. Es ist ausserdem von Vorteil für die Umsetzung, das 2-Methylpyridin mit Wasserdampf oder einem Inertgas, vorzugsweise aber mit Wasserdampf, zu verdünnen .

Das 2-Methyl-5-vinylpyrrdin kann auf einfache Weise, z.B. durch Abtrennen der Wasserphase und anschliessende Wasserdampfdestillation oder durch Vakuumdestillation so gereinigt werden, dass es für die darauffolgende Stufe b) bereitgestellt werden kann.

### Stufe b):

Die Umsetzung mit Ozon erfolgt zweckmässig in Gegenwart einer Mineralsäure bei einer Temperatur von -20 °C bis 0 °C, bevorzugt bei einer Temperatur von -15 °C bis -5 °C. Als Mineralsäure eignet sich Schwefelsäure oder Phosphorsäure, im besonderen Masse Schwefelsäure. Als Reaktionsmedium eignet sich Wasser und /oder ein polares Lösungsmittel. Als polares Lösungsmittel kann ein C₁₋₆-Alkohol wie Methanol, Ethanol, Propanol, Butanol, Pentanol oder Hexanol eingesetzt werden. Bewährt haben sich insbesondere Mischungen von einem C₁₋₆-Alkohol, wie Methanol oder Ethanol mit Wasser. Der intermediär gebildete Ozonkomplex wird zur Gewinnung des 2-Methyl-5-carbaldehyds reduktiv, bevorzugt mit einem Alkalihydrogensulfit aufgearbeitet.

Geeignete Alkalihydrogensulfite sind das Natrium- oder das Kaliumhydrogensulfit. Es ist allerdings auch möglich, andere bekannte Reduktionsmittel wie z.B. Dimethylsulfid, Thioharnstoff oder Trimethylphosphit oder Wasserstoff in Gegenwart eines geeigneten Katalysators zu wählen.

Im Falle der bevorzugten reduktiven Aufarbeitung mit Alkalihydrogensulfit arbeitet man im wesentlich gleichen Milieu wie für die Ozonisierung und üblicherweise bei einer Temperatur von -20 °C bis 20 °C, bevorzugt von -10 °C bis 0 °C.

Abhängig von den weiteren Aufarbeitungsschritten kann der 2-Methylpyridin-5-carbaldehyd oder ein Addukt von Alkalihydrogensulfit mit dem 2-Methylpyridin-5-carbaldehyd gebildet werden, nämlich ein 1-Hydroxy-(6-methylpyridin-3-yl)methansulfonsäuresalz.

Wünscht man den 2-Methyl-5-carbaldehyd zu isolieren, kann eine selektive Extraktion des Reaktionsgemisches bei einem pH von etwa 4 bis 5 mit einem geeigneten organischen Lösungsmittel, wie z. B. mit Essigsäureethylester erfolgen. Alternativ, aber bevorzugt, kann zunächst ein Addukt von Alkalihydrogensulfit mit dem 2-Methylpyridin-5-carbaldehyd gebildet werden, welches daraufhin bei einem pH-Wert von etwa 10 in den 2-Methyl-5-carbaldehyd gespalten wird.

Besonders bevorzugt wird für die Weiterumsetzung in Stufe c) jedoch gleich das Addukt von Alkalihydrogensulfit mit dem 2-Methylpyridin-5-carbaldehyd eingesetzt. Damit kann eine Isolation des relativ instabilen 2-Methylpyridin-5-carbaldehyds elegant umgangen werden.

Die Addukte von Alkalihydrogensulfit mit dem 2-Methylpyridin-5-carbaldehyd sind neu und nicht literaturbekannt und damit ebenso wie das Verfahren zu ihrer Herstellung Gegenstand der Erfindung. Die Addukte haben die allgemeine Formel 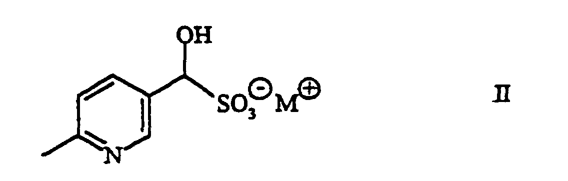 worin M ein Alkalimetall bedeutet, und werden als 1-Hydroxy-(6-methylpyridin-3-yl)methansulfonsäuresalze bezeichnet. Bevorzugte Alkalimetalle M sind Na und K.

### Stufe c):

Die Umsetzung des 2-Methylpyridin-5-carbaldehyds oder des Addukts von Alkalihydrogensulfit mit dem 2-Methylpyridin-5-carbaldehyd erfolgt nach dem Prinzip der Strecker-Synthese mit einer Cyanverbindung und einem Dialkylamin zum entsprechenden N,N-Dialkylamino-(6-methyl-3-pyridyl)acetonitril.

Als Cyanverbindung kann dabei eine wässerige HCN-Lösung oder eine wässerige Lösung von einem Alkalicyanid dienen. Besonders geeignete Dialkylamine sind C₁₋₄-Dialkylamin, worin C₁₋₄-Alkyl konkret Methyl, Ethyl, n-Propyl, iso-Propyl, n-Butyl, iso-Butyl oder tert.-Butyl bedeutet. Bevorzugte Dialkylamine sind Dimethylamin und Diethylamin.

Die Umsetzungstemperatur bewegt sich vorteilhaft im Bereich von 0 °C bis 30 °C.

Es kann von Vorteil sein, ein mit Wasser nicht mischbares Lösungsmittel, wie beispielsweise Toluol oder t-Butylmethylether, zuzusetzen. Die Aufarbeitung und Isolation des entsprechenden N,N-Dialkylamino-(6-methyl-3-pyridyl)acetonitrils kann dann über eine einfache Phasentrennung erfolgen. Die N,N-Dialkylamino-(6-methyl-3-pyridyl)acetonitrile der allgemeinen Formel 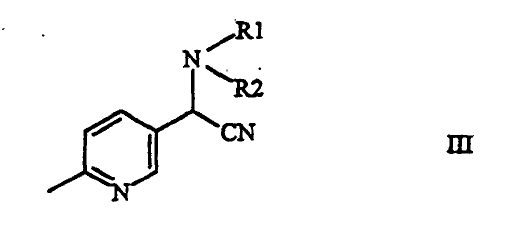 worin R¹ und R² gleich oder verschieden sind und C₁₋₄-Alkyl bedeuten, sind neue, nicht literaturbekannte Verbindungen und damit ebenso wie das Verfahren zu ihrer Herstellung Gegenstand der Erfindung.

C₁₋₄-Alkyl bedeutet wie oben Methyl, Ethyl, n-Propyl, iso-Propyl, n-Butyl, iso-Butyl oder tert.-Butyl. Bevorzugte Bedeutung von Alkyl ist Methyl oder Ethyl.

### Stufe d):

Die Überführung des N,N-Dialkylamino-(6-methyl-3-pyridyl)acetonitrils durch Reaktion mit dem 4-(Methylsulfonyl)benzylhalogenid zum Endprodukt der Formel I erfolgt in Gegenwart einer Base. Ein bevorzugtes 4-(Methylsulfonyl)benzylhalogenid ist das 4-(Methylsulfonyl)benzylchlorid.

Als Base kann dabei eine wässrige Alkalihydroxidlösung, bevorzugt eine wässrige Natriumhydroxid-Lösung eingesetzt werden, wobei in diesem Fall die Gegenwart eines üblichen Phasentransferkatalysators von Nutzen ist. Geeignete Phasentransferkatalysatoren sind z. B. die Tetraalkylammoniumhalogenide wie z. B. das Tetra-n-butylammoniumchlorid oder das Tetra-n-butylammoniumbromid . Die Umsetzungstemperatur bewegt sich dabei im Bereich von 40 °C bis 70 °C. Es kann von Vorteil sein, ein mit Wasser nicht mischbares Lösungsmittel, wie beispielsweise Toluol, Methylenchlorid oder t-Butylmethylether zuzusetzen.

Alternativ und bevorzugt kann als Base auch ein Alkalialkoholat verwendet werden. Geeignete Alkalialkoholate sind z. B. das Natrium- oder das Kalium-tert-butanolat oder das Natrium-tert-pentylat, bevorzugt aber das Kalium-tert-butanolat. Als Lösungsmittel empfehlen sich Ether wie z. B. das Tetrahydrofuran. Die Reaktionstemperatur bei dieser Variante beträgt in der Regel 15°C bis 25 °C.

Das 1-(6-Methylpyridin-3-yl)-2-[(4-(methylsulfonyl)phenyl]-ethanon kann auf fachmännisch übliche Weise, z. B. durch Ansäuern des Reaktionsgemisches und anschliessende Extraktion mit z. B. Toluol, isoliert werden. Eine weitere Aufreinigung kann durch Rekristallisation z. B. in Acetonitril, erfolgen.

### Beispiel 1

### Herstellung von 2-Methyl-5-vinylpyridin

Bimsstein mit einer Komgrösse von 6 bis 8 mm wird mit Wasser befeuchtet und mit 25% seines Trockengewichts mit Zinkoxid in Pulverform vermischt, in feuchtem Zustand in den Reaktor (Rohrlänge 750mm, Rohrdurchmesser 60mm) gefüllt und im Stickstoffstrom bei 650°C bis 700°C während 24h belassen.
76ml/h 2-Methyl-5-ethylpyridin wurden zusammen mit 87ml/h Wasserdampf bei 670°C bis 680°C und 665 mbar über den vorgenannten Katalysator geleitet. Am Reaktorende wurde ein Produktstrom bestehend aus 40,6 Gew.% 2-Methyl-5-vinylpyridin und 56,3% 2-Methyl-5-ethylpyridin entnommen. Bezogen auf umgesetztes 2-Methyl-5-ethylpyridin wurde eine Ausbeute von 93,0% erreicht.
Zur Reindarstellung des 2-Methyl-5-vinylpyridins wurde das Produktgemisch anschliessend wasserdampfdestilliert (266mbar, Kopftemperatur 59°C-60°C).
Die Reinigung von 2-Methyl-5-vinylpyridin kann auch mittels Vakuumdestillation (20 mbar, Temperatur ca. 90 °C) erfolgen.

### Beispiel 2

### Herstellung von 2-Methylpyridin-5-carbaldehyd

11,92 g 2-Methyl-5-vinylpyridin (Gehalt 85 %, 85 mmol), 50 ml Methanol und 10 ml Wasser wurden vorgelegt. Konzentrierte Schwefelsäure (9,81 g, 98 mmol) wurde so zudosiert, dass die Temperatur 20 °C nicht überstieg. Die Lösung wurde auf -12 °C gekühlt, anschliessend wurde ein Ozon/Sauerstoff Gemisch (ca. 5 % O₃. in O₂, 50 L/h) solange eingeleitet, bis das 2-Methyl-5-vinylpyridin vollständig umgesetzt wurde. Wasser (50 ml) und 40 % wässerige NaHSO₃ Lösung (22,7 g, 85 mmol) wurden vorsichtig zudosiert. Das Reaktionsgemisch wurde auf 20 °C erwärmt und mit 30 % NaOH (ca. 32 g, 0,24 mol) neutralisiert. Methanol wurde bei 30 - 40 °C abdestilliert, dann wurde zur Bildung des Bisulfit-Addukts nochmals 22,7 g 40 % NaHSO₃ Lösung zugegeben. Nach 30 min. Rühren wurde der pH wieder neutral gestellt, anschliessend wurden die neutralen Verunreinigungen mit 35 ml t-Butylmethylether extrahiert. Die wässerige Phase wurde mit 30 % NaOH auf pH 10 gebracht, und 26,5 g Na₂CO₃ (0,25 mol) wurden zugegeben. Der freigesetzte Aldehyd wurde mit 2 mal 80 ml t-Butylmethylether extrahiert. Nach Einengen des Lösungsmittels wurden 9 g 2-Methylpyridin-5-carbaldehyd als leicht gelbliches Öl erhalten.

| | |
|---|---|
| ¹H-NMR (CDCl₃) | 2,66 (s, 3H); |
| | 7,35 (d, *J* = 8 Hz, 1H); |
| | 8,07 (dd, *J* = 8 Hz and 2,1 Hz, 1 H); |
| | 8,96 (d, *J* = 2,1 Hz, 1 H); |
| | 10,08 (s, 1H). |
| | |
| ¹³C-NMR (CDCl₃) | 24,98 (CH3); |
| | 123,72 (C-5); |
| | 129,32 (C-3); |
| | 135,88 (C-4); |
| | 151,87 (C-2); |
| | 164,87 (C-6); |
| | 190,51 (C=O). |

### Beispiel 3a.

### Herstellung von N,N-Diethylamino-(6-methylpyridin-3-yl) acetonitril (ex 2-Methylpyridin-5-carbaldehyd)

73,2 g (1,25 eq.) Diethylamin und 100,3 g (1,15 eq.) einer 25 % HCN Lösung wurden bei 10 °C bis 15 °C simultan und während einer Stunde zu einer gut gerührten Mischung von 98,3 g (1,0 eq.) 2-Methylpyridin-5-carbaldehyd in 200 ml Wasser und 200 ml Toluol zugegeben. Das Reaktionsgemisch wurde während 3 h bei 30 °C gerührt.
Die Phasen wurden darauf getrennt und die Wasserphase mit 2 mal 100 ml Toluol extrahiert. Die organischen Phasen wurden zusammengenommen und dann das Toluol entfernt, worauf das Titelprodukt in Form eines gelblichen Öls und in einer Ausbeute von 172,3g (90,1 %) resultierte.

| | |
|---|---|
| ¹H-NMR (CDCl₃) | 8,65 (1H, s); |
| | 7,75 (1H, d); |
| | 7,20 (1H, d); |
| | 5,00 (1H, s); |
| | 2,68 (2H, m); |
| | 2,59 (13H, s); |
| | 2,50 (2H, m); |
| | 1,10 (6H, t). |
| | |
| ¹H-NMR (D₆-DMSO) | 8,50 (1H, s); |
| | 7,70 (1H, d); |
| | 7,32 (1H, d); |
| | 5,45 (1H, s); |
| | 2,58 (2H, m); |
| | 2,50 (3H, s); |
| | 2,40 (2H, m); |
| | 1,02 (6H, t). |

### Beispiel 3b.

### Herstellung von N,N-Diethylamino-(6-methylpyridin-3-yl) acetonitril (via Addukt von 2-Methylpyridin-5-carbaldehyd mit Natriumhydrogensulfit)

Die Ozonolyse wurde durchgeführt wie in Beispiel 2, ausgehend von 23,84 g 2-Methyl-5-vinylpyridine (83,1 % GC, 166,2 mmol). Nachdem die Verunreinigungen bei neutralem pH extrahiert worden waren, wurde die wässerige Phase auf 15 °C gekühlt und 21,94 g Diethylamin (0,3 mol), dann 9,8 g NaCN (0,2 mol) zugegeben (je 10 Min. Zugabezeit). Die Lösung wurde 4,5 h bei 15 °C gerührt und das Produkt wurde anschliessend mit 3 mal 85 ml Toluol extrahiert.

Die vereinigten Extrakte wurden eingeengt. Erhalten: 37,4 g N,N-Diethylamino-(6-methylpyridin-3-yl)acetonitril als oranges Öl. Gehalt : 83,7 % (GC, Gewicht-%), 0,34 % Aldehyd). Ausbeute: 92,7 % bezogen auf 2-Methyl-5-vinylpyridin.

| | |
|---|---|
| ¹H-NMR (CDCl₃) | 1,08 (t, 6H); |
| | 2,50 (m, 2H); |
| | 2,58 (s, 3H); |
| | 2,65 (m, 2H); |
| | 5,00 (s, 1H); |
| | 7,18 (d, *J*= 8 Hz, 1H); |
| | 7,74 (dd, *J*= 8 Hz, 2 Hz, 1H); |
| | 8,66 (d, *J*= 2 Hz, 1H). |

### Beispiel 3c.

### Herstellung und Charakterisierung des Addukts von 2-Methylpyridin-5-carbaldehyd mit Natriumhydrogensulfit

Nach der Bisulfit-Zugabe wurde eine Probe mit ¹H- und ¹³C-NMR gemessen. Die NMR-Signale des Aldehyds waren total verschwunden, stattdessen wurden die folgenden Signale beobachtet:

| | |
|---|---|
| ¹H-NMR (DMSO-d₆) | 1,96 (s, 3H); |
| | 5,01 (s, 1H); |
| | 6,85 (d, *J* = 8 Hz, 1H); |
| | 7,45 (dd, *J*= 8 and 2 Hz, 1H); |
| | 7,93 (d, *J*= 2 Hz, 1H). |
| | |
| ¹³C-NMR (DMSO-d₆) | 20,23 (CH3); |
| | 81,78 (CH); |
| | 124,14 (C-5); |
| | 130,02 (C-3); |
| | 138,76 (C-4); |
| | 143,08 (C-2); |
| | 156,04 (C-6). |

### Beispiel 4a.

### Herstellung von 1-(6-Methylpyridin-3-yl)-2-[(4-(methylsulfonyl)phenyl]-ethanon (wässrige NaOH als Base)

41,07g (89,1%, 1,00eq.) N,N-Diethylamino-(6-methylpyridin-3-yl) acetonitril, 30ml Toluol und 10,0g Cellite wurden vorgelegt. 72g (5eq.) einer 50% wässrigen NaOH-Lösung wurden darauf während 15 Minuten so zugegeben, dass die Temperatur auf 20°C gehalten werden konnte. Das Reaktionsgemisch wurde auf 45°C erwärmt. Unter starkem Rühren wurde eine erste Portion von 0,32g Tetra-n-butylammoniumbromid zugegeben.
Unmittelbar darauf gab man während 1,5h eine Lösung 0,32g Tetra-n-butylammoniumbromid und 44,52g (1,2eq.) 4-(Methylsulfonyl)benzylchlorid in 200ml Toluol zu. Nach der Hälfte der Zugabe wird eine dritte Portion von 0,32g Tetra n-butyl ammonium bromid zugegeben und während 6h bei 45°C weitergerührt.
Das Reaktionsgemisch wurde darauf auf Raumtemperatur gebracht, dann wurden 100ml Wasser und 100ml Toluol zugegeben. Nach Filtration und Waschen des Rückstands mit 25 ml Toluol wurden die Phasen getrennt. Die Wasserphase wurde mit 2 mal 50 ml Toluol extrahiert. Die kombinierten organischen Phasen wurden darauf mit 380 ml 1N HCl extrahiert. Neutralisation mit 29,6g 50% wässriger NaOH-Lösung bis pH 4,5 führte zur Auskristallisation des Titelprodukts. Die Suspension wurde filtriert, das Produkt mit 2 mal 100ml Wasser und 2 mal 80 ml Isopropanol/Wasser 1:1 gewaschen und anschliessend bei 20°C / 20mbar getrocknet.
Man erhielt 40,19 g (76,4%) des Titelproduktes mit einem Gehalt von 99,0%.
Fp. 182°C-183°C

| | |
|---|---|
| ¹H-NMR(CDCl₃) | 9,15 (1H, s); |
| | 8,18 (1H, d); |
| | 7,92 (2H, d); |
| | 7,47 (2H, d); |
| | 7,30 (1H, d); |
| | 4,39 (2H, s); |
| | 3,04 (3H, s); |
| | 2,63 (3H, s). |

### Beispiel 4b.

### Herstellung von 1-(6-Methylpyridin-3-yl)-2-[(4-(methylsulfonyl)phenyl]ethanon (Alkoholat, wasserfrei)

48,16g (84,5%, 1,00eq.) N,N-Diethylamino-(6-methylpyridin-3-yl) acetonitril in 20 ml Tetrahydrofuran wurden während 30 Minuten bei 20°C zu einer Suspension von 38,58g (1,7 eq.) Kalium-t-butanolat in 60 ml Tetrahydrofuran zugegeben. Unmittelbar anschliessend wurden 42,59g (1,03 eq) 4-(Methylsulfonyl)benzylchlorid in 60 ml Tetrahydrofuran während 1,5h bei 20°C bis 25°C zugegeben.
Nach 0,5h Rühren bei 20°C wurde das Reaktionsgemisch mit 100ml Wasser verdünnt und mit 180ml 2N HCl während einer Stunde auf pH 2 gebracht. Nach weiteren 0,5h bei 20°C wurde mit 10g einer 30% wässrigen NaOH-Lösung auf pH 3 gestellt. Nach einer Stunde Rühren bei 20 °C wurde die Suspension filtriert, das Produkt mit 2 mal 150 ml Wasser und 2 mal 100 ml Wasser/Isopropanol 1:1 gewaschen. Nach dem Trocknen bei 20°C/20mbar wurden 53,72g (92%) des Titelproduktes mit einem Gehalt von 99,1% erhalten.
Fp. 182°C-183°C

| | |
|---|---|
| ¹H-NMR (CDCl₃) | 9,15 (1H, s); |
| | 8,18 (1H, d); |
| | 7,92 (2H, d); |
| | 7,47 (2H, d); |
| | 7,30 (1H, d); |
| | 4,39 (2H, s); |
| | 3,04 (3H, s); |
| | 2,63 (3H, s). |

## Patentansprüche

1. Verfahren zur Herstellung von 1-(6-Methylpyridin-3-yl)-2-[(4-(methylsulfonyl)phenyl]-ethanon der Formel 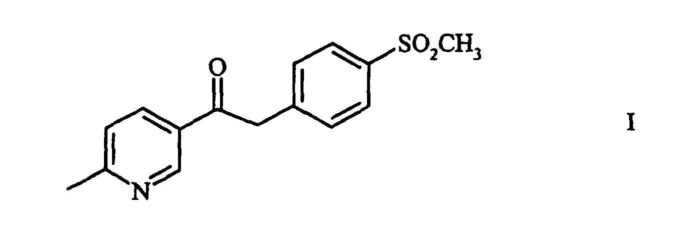 **dadurch gekennzeichnet, dass** N,N-Dialkylamino-(6-methyl-3-pyridyl)acetonitril, wobei die beiden Alkylreste C1-C4-Alkyl sind und gleich oder verschieden sind, in Gegenwart einer Base mit einem 4-(Methylsulfonyl)benzylhalogenid zum 1-(6-Methylpyridin-3-yl)-2-[(4-(methylsulfonyl)phenyl]-ethanon reagiert, wobei
als Base ein Alkalialkoholat in Gegenwart eines organischen Lösungsmittels bei einer Reaktionstemperatur von 15 °C bis 25 °C verwendet wird.

2. Verfahren gemäss Patentanspruch 1, **dadurch gekennzeichnet, dass** das 4-(methylsulfonyl)benzylhalogenid 4-(Methylsulfonyl)benzylchlorid ist.

3. Verfahren gemäss einem der Patentansprüche 1 und 2, **dadurch gekennzeichnet, dass** das Acetonitril zu einer Suspension des wasserfreien Alkalialkoholates zugegeben wird.

4. Verfahren gemäss einem der Patentansprüche 1 bis 3, **dadurch gekennzeichnet, dass** das Alkalialkoholat ausgewählt ist aus der Gruppe umfassend Natrium tert-Butanolat, Kalium tert-Butanolat und Natrium tert-Pentylat.

5. Verfahren gemäss Anspruch 4, **dadurch gekennzeichnet, dass** das Alkalialkoholat Kalium tert-Butanolat ist.

6. Verfahren gemäss Anspruch 1 bis 5, **dadurch gekennzeichnet, dass** das organische Lösungsmittel ein Ether ist.

7. Verfahren gemäss Anspruch 6, **dadurch gekennzeichnet, dass** der Ether Tetrahydrofuran ist.

## Claims

1. A process for preparing 1-(6-methylpyridin-3-yl)-2-[(4-(methylsulfonyl)phenyl]-ethanone of the formula 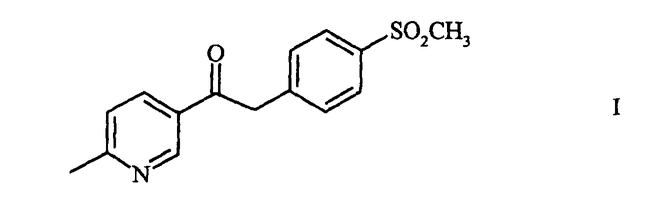 **characterized in that** N,N-dialkylamino-(6-methyl-3-pyridyl)acetonitrile, wherein the alkyl radicals are C₁-C₄ alkyl and are identical or different, is reacted in the presence of a base with a 4-(methylsulfonyl)benzyl halogenide to give 1-(6-methylpyridin-3-yl)-2-[(4-(methylsulfonyl)phenyl]ethanone, wherein the base is an alkali alcoholate and is used in the presence of an organic solvent at a temperature of 15°C to 25°C.

2. Process according to claim 1, **characterized in that** the 4-(methylsulfonyl)benzyl halogenide is 4-(methylsulfonyl)benzyl chloride.

3. Process according to claim 1 or 2, **characterized in that** the acetonitrile is added to a water-free suspension of the alkali alcoholate

4. Process according to any of claims 1 to 3, **characterized in that** the alkali alkoholat is selected from a group comprising sodium tert-butanolate, potassium tert-butanolate and sodium tert-pentanolate.

5. Process according to claim 4, **characterized in that** the alkali alcoholat is potassium tert-butanolate.

6. Process according to claims 1 to 5, **characterized in that** the organic solvent is an ether.

7. Process according to claim 6, **characterized in that** the ether is tetrahydrofuran.

## Revendications

1. Procédé pour la préparation de 1-(6-méthylpyridin-3-yl)-2-[4-(méthylsulfonyl)phényl]éthanone de formule 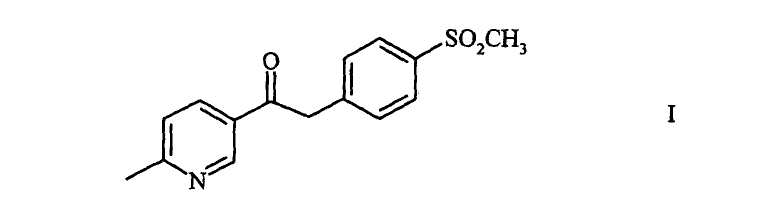 **caractérisé en ce qu'**on fait réagir un N,N-dialkylamino-(6-méthyl-3-pyridyl)acétonitrile, dans lequel les deux radicaux alkyle sont des groupes alkyle en C₁-C₄ et sont identiques ou différents, en présence d'une base, avec un halogénure de 4-(méthylsulfonyl)benzyle, pour aboutir à la 1-(6-méthylpyridin-3-yl)-2-[4-(méthylsulfonyl)phényl]éthanone, en utilisant en tant que base un alcoolate de métal alcalin en présence d'un solvant organique, à une température de réaction de 15°C à 25°C.

2. Procédé selon la revendication 1, **caractérisé en ce que** l'halogénure de 4-(méthylsulfonyl)benzyle est le chlorure de 4-(méthylsulfonyl)benzyle.

3. Procédé selon la revendication 1 ou 2, **caractérisé en ce qu'**on ajoute l'acétonitrile à une suspension de l'alcoolate de métal alcalin anhydre.

4. Procédé selon l'une quelconque des revendications 1 à 3, **caractérisé en ce que** l'alcoolate de métal alcalin est choisi dans le groupe comprenant le tert-butanolate de sodium, le tert-butanolate de potassium et le tert-pentylate de sodium.

5. Procédé selon la revendication 4, **caractérisé en ce que** l'alcoolate de métal alcalin est le tert-butanolate de potassium.

6. Procédé selon les revendications 1 à 5, **caractérisé en ce que** le solvant organique est un éther.

7. Procédé selon la revendication 6, **caractérisé en ce que** l'éther est le tétrahydrofuranne.
